(19) Europäisches Patentamt
European Patent Office
Office européen des brevets

(11) EP 2 309 564 A1

(12) **EUROPEAN PATENT APPLICATION**
published in accordance with Art. 153(4) EPC

(43) Date of publication:
**13.04.2011 Bulletin 2011/15**

(21) Application number: **09770138.7**

(22) Date of filing: **23.06.2009**

(51) Int Cl.:
***H01L 51/50** (2006.01)* ***C07D 403/04** (2006.01)*
***C07D 403/14** (2006.01)* ***C07F 15/00** (2006.01)*
***C08G 61/12** (2006.01)* ***C08G 73/06** (2006.01)*
***C08K 5/34** (2006.01)* ***C08K 5/56** (2006.01)*
***C08L 65/00** (2006.01)* ***C09K 11/06** (2006.01)*

(86) International application number:
**PCT/JP2009/061365**

(87) International publication number:
**WO 2009/157428 (30.12.2009 Gazette 2009/53)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **23.06.2008 JP 2008163042**

(71) Applicants:
- **Sumitomo Chemical Company, Limited**
**Tokyo 104-8260 (JP)**
- **Sumation Co., Ltd.**
**Chuo-ku**
**Tokyo 104-8260 (JP)**

(72) Inventor: **AKINO Nobuhiko**
**Tsukuba-shi**
**Ibaraki 305-0047 (JP)**

(74) Representative: **Duckworth, Timothy John**
**J.A. Kemp & Co.**
**14 South Square**
**Gray's Inn**
**London WC1R 5JJ (GB)**

# (54) PHOSPHORESCENT LIGHT-EMITTING COMPOSITION AND LIGHT-EMITTING ELEMENT COMPRISING THE COMPOSITION

(57) Disclosed is a composition comprising: a compound having a residue of at least one nitrogenated compound selected from the group consisting of nitrogenated compounds represented by formulae (1-1), (1-2), (1-3) and (1-4) [wherein R's independently represent a hydrogen atom or a substituent and may be the same as or different from one another] and a residue of at least one nitrogenated polycyclic compound selected from the group consisting of nitrogenated polycyclic compounds represented by formulae (2-1), (2-2), (2-3) and (2-4) [wherein R's are as defined above]; and a phosphorescent light-emitting compound.

EP 2 309 564 A1

## Description

### TECHNICAL FIELD

The present invention relates to a phosphorescent composition and a light-emitting device prepared by using the composition.

### BACKGROUND ART

As a light-emitting material for use in a light-emitting layer of a light-emitting device, a compound emitting light from a triplet excitation state (hereinafter, sometimes referred to as a "phosphorescent compound") is known. The device using this compound in a light-emitting layer is known to have a high luminous efficiency. When a phosphorescent compound is used in a light-emitting layer, usually, a composition prepared by adding the compound to a matrix is used as a light-emitting material. As the matrix, polyvinylcarbazole is used since a thin film can be formed by coating (PATENT DOCUMENT 1).

However, it is difficult to inject electrons to this compound because the energy level of the lowest unoccupied molecular orbital (hereinafter, referred to as the "LUMO") thereof is high. On the other hand, a conjugated polymer compound such as polyfluorene has a low LUMO. Thus, if it is used as a matrix, a low driving voltage can be realized relatively easily. However, such a conjugated polymer compound, since the lowest triplet excitation energy thereof is low, is not suitable as a matrix used for emitting light having a shorter wavelength than that of green light, in particular (PATENT DOCUMENT 2). For example, in a light-emitting material composed of polyfluorene as a conjugated polymer compound and a triplet emission compound (NON-PATENT DOCUMENT 1), light emission from triplet emission compound is weak. Thus, the luminous efficiency thereof is low.

### CITATION LIST

### PATENT DOCUMENTS

PATENT DOCUMENT 1: JP 2002-50483 A
PATENT DOCUMENT 2: JP 2002-241455 A

### NON-PATENT DOCUMENT

NON-PATENT DOCUMENT 1: APPLIED PHYSICS LETTERS, 80, 13, 2308 (2002)

### SUMMARY OF THE INVENTION

### PROBLEMS TO BE SOLVED BY THE INVENTION

In the circumstances, an object of the invention is to provide a light-emitting material providing an excellent luminous efficiency when used in a light-emitting device, etc.

### MEANS FOR SOLVING THE PROBLEMS

The present invention firstly provides a composition containing: a compound having a residue(s) of at least one nitrogen-containing compound selected from the group consisting of nitrogen-containing compounds represented by the following formulas (1-1), (1-2), (1-3) and (1-4):

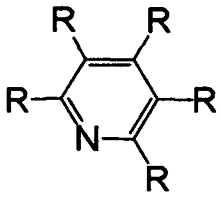
(1-1)

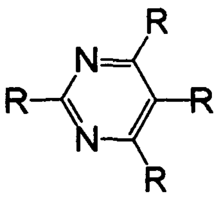
(1-2)

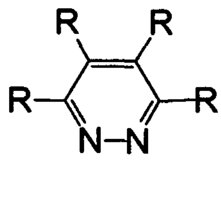
(1-3)

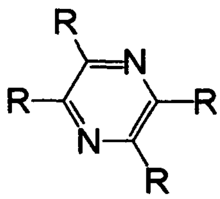
(1-4)

wherein R represents a hydrogen atom or a substituent; and more than one R may be the same or different, and a residue(s) of at least one nitrogen-containing polycyclic compound selected from the group consisting of nitrogen-containing polycyclic compounds represented by the following formulas (2-1), (2-2), (2-3) and (2-4):

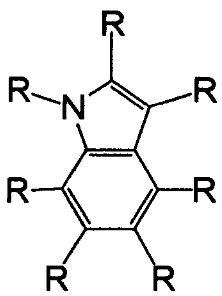
(2-1)

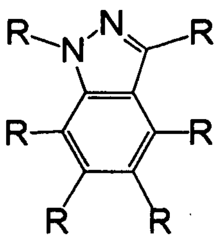
(2-2)

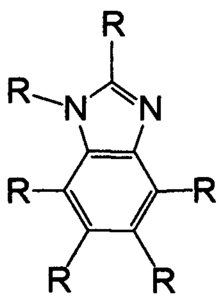
(2-3)

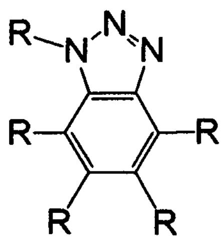
(2-4)

wherein R is as defined above;
and a phosphorescent compound.
The present invention secondly provides a polymer compound: containing a residue(s) of at least one nitrogen-containing compound selected from the group consisting of nitrogen-containing compounds represented by the above formulas (1-1), (1-2), (1-3) and (1-4); a residue(s) of at least one nitrogen-containing polycyclic compound selected from the group consisting of nitrogen-containing polycyclic compounds represented by the above formulas (2-1), (2-2), (2-3) and (2-4); and a residue(s) of a phosphorescent compound.
The present invention thirdly provides a film and a light-emitting device prepared by using the composition or the polymer compound.
The present invention provides fourthly a planar light source, a display and light having the light-emitting device.

## ADVANTAGES OF THE INVENTION

The composition and polymer compound of the present invention (hereinafter, referred to as "the composition, etc. of the present invention") have a high luminous efficiency. Therefore, when the composition, etc. of the present invention, are used in preparation of a light-emitting device, etc., it serves as a light-emitting material excellent in luminous efficiency. Furthermore, the composition, etc. of the present invention usually have a relatively excellent light emitting property when they emit light in a relatively short wavelength region. This is because a compound having a residue(s) of a nitrogen-containing compound and a residue(s) of a nitrogen-containing polycyclic compound contained in the composition of the present invention and the polymer compound of the present invention have large $T_1$ energy values. In addition, the LUMO energy level is relatively low and thus electrons are easily injected, and the energy level of the highest occupied molecular orbital (hereinafter, referred to as the "HOMO") is relatively high and thus holes are easily injected.

## MODE FOR CARRYING OUT THE INVENTION

Next, the present invention will be more specifically described below. Note that in the specification, in the case where an alkyl group and an alkoxy group of a structural formula has no prefix (t-, etc.), they means n-.

<Composition>

The composition of the present invention is a composition containing: a compound (hereinafter, sometimes referred to as the "compound having a residue(s) of a nitrogen-containing compound and a residue(s) of a nitrogen-containing polycyclic compound") having a residue(s) of at least one nitrogen-containing compound selected from the group consisting of nitrogen-containing compounds represented by the above formulas (1-1), (1-2), (1-3) and (1-4) (hereinafter, referred to as "formulas (1-1) to (1-4)") and a residue(s) of at least one nitrogen-containing polycyclic compound selected from the group consisting of nitrogen-containing polycyclic compounds represented by the above formulas (2-1), (2-2), (2-3) and (2-4) (hereinafter, referred to as "formulas (2-1) to (2-4)"); and a phosphorescent compound. In the present invention, for example, the residues of compounds represented by the above formulas (1-1) to (1-4) refer to groups provided by removing all or some (in particular 1 to 3 R) of the R from the respective compounds represented by the above formulas (1-1) to (1-4); whereas the residues of compounds represented by the above formulas (2-1) to (2-4) refer to groups provided by removing all or some (in particular 1 to 3 R) of the R from respective compounds represented by the above formulas (2-1) to (2-4). Furthermore, the "polymer compound" refers to a compound having at least two identical structures (repeating units) therein.

In the compound having a residue(s) of a nitrogen-containing compound and a residue(s) of a nitrogen-containing polycyclic compound, as the residue(s) of a nitrogen-containing compound, a residue(s) of a nitrogen-containing compound selected from the group consisting of nitrogen-containing compounds represented by the above formulas (1-2), (1-3) and (1-4) is preferable.

In the compound having a residue(s) of a nitrogen-containing compound and a residue(s) of a nitrogen-containing polycyclic compound, a residue(s) of a nitrogen-containing polycyclic compound, as the residue(s) of a nitrogen-containing polycyclic compound, a residue(s) of at least one nitrogen-containing polycyclic compound selected from the group consisting of nitrogen-containing polycyclic compounds represented by the above formulas (2-1), (2-2) and (2-3) is preferable.

As the compound having a residue(s) of a nitrogen-containing compound and a residue(s) of a nitrogen-containing polycyclic compound, a compound having a residue(s) of at least one nitrogen-containing compound selected from the group consisting of nitrogen-containing compounds represented by the above formulas (1-2), (1-3) and (1-4), a residue(s) of at least one nitrogen-containing polycyclic compound selected from the group consisting of nitrogen-containing polycyclic compounds represented by the above formulas (2-1), (2-2) and (2-3) is preferable.

The compound having a residue(s) of a nitrogen-containing compound and a residue(s) of a nitrogen-containing polycyclic compound may be a polymer compound. In this case, a polymer compound preferably has a residue(s) of the nitrogen-containing compound and the nitrogen-containing polycyclic compound in the main chain and/or a side chain, and a polymer compound having a repeating unit containing a residue(s) of a nitrogen-containing compound represented by any one of the above formulas (1-1) to (1-4) and a residue(s) of a nitrogen-containing polycyclic compound represented by any one of the above formulas (2-1) to (2-4), and a polymer compound having a repeating unit containing any one of the structures selected from an aromatic ring, a heterocyclic ring having at least 5-members containing a hetero atom, aromatic amine and a structure represented by a formula (4) described later, in addition to a repeating unit containing a residue(s) of a nitrogen-containing compound represented by any one of the above formulas (1-1) to (1-4) and a residue(s) of a nitrogen-containing polycyclic compound represented by any one of the above formulas (2-1) to (2-4), are particularly preferable.

In the above formulas (1-1) to (1-4) and (2-1) to (2-4), R represents a hydrogen atom or a substituent, preferably, at least one of more than one R is a substituent, more preferably, at least two of the more than one R are substituents, and further preferably, all R are substituents. If there is more than one R, they may be the same or different.

Examples of the substituent include a halogen atom, an alkyl group, an alkoxy group, an alkylthio group, an aryl group that may have a substituent, an aryloxy group, an arylthio group, an arylalkyl group, an arylalkyloxy group, an arylalkylthio group, an acyl group, an acyloxy group, an amide group, an acid imide group, an imine residue, a substituted amino group, a substituted silyl group, a substituted silyloxy group, a substituted silylthio group, a substituted silylamino group, a monovalent heterocyclic group that may have a substituent, a heteroaryl group that may have a substituent, a heteroaryloxy group, a heteroarylthio group, an arylalkenyl group, an arylethynyl group, a substituted carboxyl group and a cyano group, and preferably, include an alkyl group, an alkoxy group, an aryl group that may have a substituent and a heteroaryl group that may have a substituent. Note that the N-valent heterocyclic group (N is 1 or 2) refers to a remaining atomic group provided by removing N hydrogen atoms from a heterocyclic compound; the same applies hereinafter. Note that as a monovalent heterocyclic group, a monovalent aromatic heterocyclic group is preferable.

At least one of the R is preferably an alkyl group, an alkoxy group, an aryl group that may have a substituent or a heteroaryl group that may have a substituent. At least one of the R is further preferably an alkyl group having 3 to 10 carbon atoms or an alkoxy group having 3 to 10 carbon atoms.

At least one of the R is preferably a substituent having 3 or more atoms in total except a hydrogen atom, further preferably a substituent having 5 or more atoms in total except a hydrogen atom, and particularly preferably a substituent

having 7 or more atoms in total except a hydrogen atom. When two R are present, at least one of the R is preferably a substituent, and more preferably two R are substituents. More than one R may be the same or different.

Examples of the compound having a residue(s) of a nitrogen-containing compound and a residue(s) of a nitrogen-containing polycyclic compound include a compound represented by the following formula (A-1) or (A-2):

$$Z^1 - (Y^1)_m - Z^2 \qquad (A\text{-}1)$$

$$Z^1 - (Y^2)_n - Z^2 \qquad (A\text{-}2)$$

wherein one of $Z^1$ and $Z^2$ represents a residue(s) of a nitrogen-containing compound represented by the above formula (1-1), (1-2), (1-3) or (1-4) and the other represents a residue(s) of a nitrogen-containing polycyclic compound represented by the above formula (2-1), (2-2), (2-3) or (2-4); $Y^1$ represents $-C(R^a)(R^b)-$, $-N(R^c)-$, -O-, $-Si(R^d)(R^e)-$, $-P(R^f)-$ or -S-; $R^a$ to $R^f$ each independently represent a hydrogen atom or a substituent; m is an integer of 0 to 5; when there is more than one $Y^1$, they may be the same or different; $Y^2$ represents an arylene group that may have a substituent; n is an integer of 1 to 5; and when there is more than one $Y^2$, they may be the same or different, and a compound having a residue of the foregoing compound.

Examples of the substituents represented by $R^a$ to $R^f$ include an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group an amino group, a substituted amino group, a silyl group, a substituted silyl group, a silyloxy group, a substituted silyloxy group, a monovalent heterocyclic group and a halogen atom.

Examples of the aryl group represented by $R^a$ to $R^f$ include a phenyl group, a $C_1$ to $C_{12}$ alkoxyphenyl group ("$C_1$ to $C_{12}$ alkoxy" means that the number of carbon atoms of the alkoxy moiety is 1 to 12. The same applies hereinafter), a $C_1$ to $C_{12}$ alkylphenyl group ("$C_1$ to $C_{12}$ alkyl" means that the number of carbon atoms in the alkyl moiety is 1 to 12. The same applies hereinafter), a 1-naphthyl group, a 2-naphthyl group and a pentafluorophenyl group, and preferably include a phenyl group, a $C_1$ to $C_{12}$ alkoxyphenyl group and a $C_1$ to $C_{12}$ alkylphenyl group.

The monovalent heterocyclic group represented by $R^a$ to $R^f$ refers to the remaining atomic group provided by removing a single hydrogen atom from a heterocyclic compound. The heterocyclic compound herein refers to an organic compound having a cyclic structure and containing not only carbon atoms but also hetero atoms such as an oxygen atom, a sulfur atom, a nitrogen atom and a phosphorus atom as elements constituting the ring.

When the compound having a residue(s) of a nitrogen-containing compound and a residue(s) of a nitrogen-containing polycyclic compound is a polymer compound, the compound is preferably a polymer compound having a repeating unit containing a residue(s) of a compound represented by the above formula (A-1) or (A-2), in view of $T_1$ energy.

Furthermore, the compound having a residue(s) of a nitrogen-containing compound and a residue(s) of a nitrogen-containing polycyclic compound, in view of $T_1$ energy, preferably has also a residue(s) of a compound represented by the following formula (A-3):

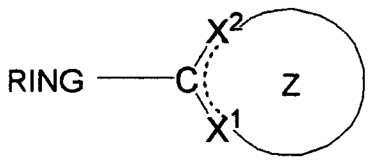

(A-3)

wherein RING refers to a residue of a compound having a residue(s) of at least one nitrogen-containing compound selected from the group consisting of nitrogen-containing compounds represented by the above formulas (1-1) to (1-4) and a residue(s) of at least one nitrogen-containing polycyclic compound selected from the group consisting of nitrogen-containing polycyclic compounds represented by the above formula (2-1) to (2-4); Ring Z represents a cyclic structure containing a carbon atom, $X^1$ and $X^2$; $X^1$ and $X^2$ each independently represent - C(R)=; and R is as defined above.

In the above formula (A-3), as the cyclic structure, an aromatic ring that may have a substituent and a non-aromatic ring that may have a substituent are mentioned. Preferable examples thereof include a benzene ring, a heterocyclic ring, an alicyclic hydrocarbon ring, a ring formed by condensing these rings and these rings whose hydrogen

atoms are partly substituted.

The residues of compounds represented by the above formulas (A-1) to (A-3) each refer to a group provided by removing all or some of the hydrogen atoms and R of the compound.

The compound having a residue(s) of a nitrogen-containing compound and a residue(s) of a nitrogen-containing polycyclic compound, whose energy level can be controlled by using a residue(s) of a nitrogen-containing compound and a residue(s) of a nitrogen-containing polycyclic compound having different HOMO/LUMO, is excellent in charge injection/transport property. Furthermore, in view of symmetry of the compound structure, amorphous nature can be improved and film formation property can be also improved.

The compound having a residue(s) of a nitrogen-containing compound and a residue(s) of a nitrogen-containing polycyclic compound may contain another type of partial structure. A preferable type of partial structure differs depending upon whether it is present at an end or not.

When another partial structure is not present at an end, a polyvalent group having a conjugating property is preferable in view of an LUMO energy level. Examples of such a group include a divalent aromatic group and a trivalent aromatic group. The aromatic group herein refers to a group derived from an aromatic organic compound. Examples of such an aromatic group include groups provided by replacing n' (n' is 2 or 3) hydrogen atoms of an aromatic ring, such as benzene, naphthalene, anthracene, pyridine, quinoline and isoquinoline, by bonds.

Examples of another preferable partial structure that may be included in the compound having a residue(s) of a nitrogen-containing compound and a residue(s) of a nitrogen-containing polycyclic compound include a structure represented by the following formula (4):

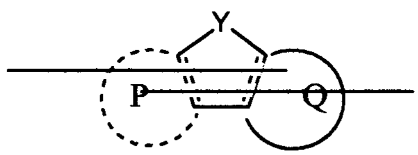

(4)

In the above formula (4), ring P and ring Q may have a substituent selected from the group consisting of an alkyl group, an alkoxy group, an alkylthio group, an aryl group, an alkenyl group, an alkynyl group, an aryloxy group, an arylthio group, an arylalkyl group, an arylalkoxy group, an arylalkylthio group, an arylalkenyl group, an arylalkynyl group, an amino group, a substituted amino group, a silyl group, a substituted silyl group, a halogen atom, an acyl group, an acyloxy group, an imine residue, an amide group, an acid imide group, a monovalent heterocyclic group, a carboxyl group, a substituted carboxyl group and a cyano group. As the substituent, a substituent selected from the group consisting of an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, an amino group, a substituted amino group, a silyl group, a substituted silyl group, a halogen atom, an acyl group, an acyloxy group, an imine residue, an amide group, an acid imide group, a monovalent heterocyclic group, a carboxyl group, a substituted carboxyl group and a cyano group is preferable.

In the above formula (4), ring P and ring Q each independently represent an aromatic ring; however, ring P may exist or not. When ring P is present, two bonds are present one on ring P and one on ring Q. When ring P is not present, two bonds are present one on a 5-membered ring or 6-membered ring including Y and one on ring Q. Furthermore, on ring P, ring Q or a 5-membered ring or 6-membered ring including Y, a substituent may be present, which is selected from the group consisting of an alkyl group, an alkoxy group, an alkylthio group, an aryl group, an alkenyl group, an alkynyl group, an aryloxy group, an arylthio group, an arylalkyl group, an arylalkoxy group, an arylalkylthio group, an arylalkenyl group, an arylalkynyl group, an amino group, a substituted amino group, a silyl group, a substituted silyl group, a halogen atom, an acyl group, an acyloxy group, an imine residue, an amide group, an acid imide group, a monovalent heterocyclic group, a carboxyl group, a substituted carboxyl group and a cyano group. As the substituent, a substituent selected from the group consisting of an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, an amino group, a substituted amino group, a silyl group, a substituted silyl group, a halogen atom, an acyl group, an acyloxy group, an imine residue, an amide group, an acid imide group, a monovalent heterocyclic group, a carboxyl group, a substituted carboxyl group and a cyano group is preferable. Y represents -O-, -S-, -Se-, -B($R^0$)-, -Si($R^2$)($R^3$)-, -P($R^4$)-, -P($R^5$)(=O)-, -C($R^6$)($R^7$)-, -N($R^8$)-, -C($R^9$)($R^{10}$)-C($R^{11}$)($R^{12}$)-, -O-C($R^{13}$)($R^{14}$)-, -S-C($R^{15}$)($R^{16}$)-, -N-C($R^{17}$)($R^{18}$)-, -Si($R^{19}$)($R^{20}$)-C($R^{21}$)($R^{22}$)-, -Si($R^{23}$)($R^{24}$)-Si($R^{25}$)($R^{26}$)-, -C($R^{27}$) = C($R^{28}$)-, -N = C($R^{29}$)-, or -Si($R^{30}$) = C($R^{31}$)-. $R^0$ and $R^2$ to $R^{31}$ herein each independently represent a hydrogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, an amino group, a substituted amino group, a silyl group,

a substituted silyl group, a silyloxy group, a substituted silyloxy group, a monovalent heterocyclic group or a halogen atom. Of them, a hydrogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkenyl group, an amino group, a substituted amino group, a silyl group, a substituted silyl group, a silyloxy group, a substituted silyloxy group, a monovalent heterocyclic group and a halogen atom are preferable; an alkyl group, an alkoxy group, an alkylthio group, an aryl group, an aryloxy group, an arylthio group, an arylalkyl group, an arylalkoxy group and a monovalent heterocyclic group are more preferable; an alkyl group, an alkoxy group, an aryl group and a monovalent heterocyclic group are further preferable; and an alkyl group and an aryl group are particularly preferable.

Examples of the structure represented by the above formula (4) include a structure represented by the following formula (4-1), (4-2) or (4-3):

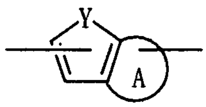
(4-1)

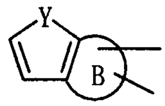
(4-2)

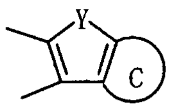
(4-3)

wherein ring A, ring B and ring C each independently represent an aromatic ring; formulas (4-1), (4-2) and (4-3) each may have a substituent selected from the group consisting of an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, an amino group, a substituted amino group, a silyl group, a substituted silyl group, a halogen atom, an acyl group, an acyloxy group, an imine residue, an amide group, an acid imide group, a monovalent heterocyclic group, a carboxyl group, a substituted carboxyl group and a cyano group; and Y is as defined above,
and a structure represented by the following formula (4-4) or (4-5):

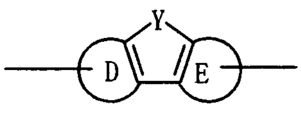
(4-4)

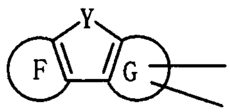
(4-5)

wherein ring D, ring E, ring F and ring G each independently represent an aromatic ring that may have a substituent selected from the group consisting of an alkyl group, an alkoxy group, an alkylthio group, an aryl group, an aryloxy group, an arylthio group, an arylalkyl group, an arylalkoxy group, an arylalkylthio group, an arylalkenyl group an arylalknyl group, an amino group, a substituted amino group, a silyl group, a substituted silyl group, a halogen atom, an acyl group, an acyloxy group, an imine residue, an amide group, an acid imide group, a monovalent heterocyclic group, a carboxyl group, a substituted carboxyl group and a cyano group; and Y is as defined above.

In the formulas (4-1), (4-2), (4-3), (4-4) and (4-5), examples of aromatic rings represented by ring A, ring B, ring C, ring D, ring E, ring F and ring G and having no substituents include aromatic hydrocarbon rings such as a benzene ring, a naphthalene ring, an anthracene ring, a tetracene ring, a pentacene ring, a pyrene ring and a phenanthrene ring; and heteroaromatic rings such as a pyridine ring, a bipyridine ring, a phenanthroline ring, a quinoline ring, an isoquinoline ring, a thiophene ring, a furan ring and a pyrrole ring. These aromatic rings may have the aforementioned substituents.

Furthermore, examples of another preferable partial structure that may be contained in the compound having a residue(s) of a nitrogen-containing compound and a residue(s) of a nitrogen-containing polycyclic compound include an aromatic amine structure represented by the following formula:

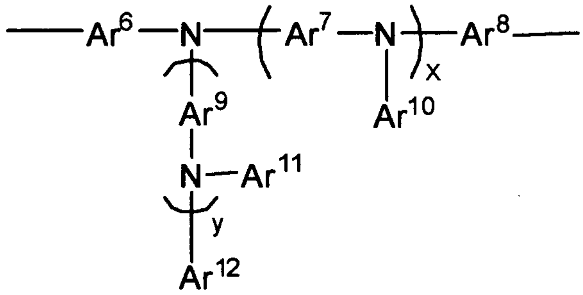

wherein $Ar^6$, $Ar^7$, $Ar^8$ and $Ar^9$ each independently represent an arylene group or a divalent heterocyclic group; $Ar^{10}$, $Ar^{11}$ and $Ar^{12}$ each independently represent an aryl group or a monovalent heterocyclic group; $Ar_6$ to $Ar_{12}$ may have a substituent; and x and y each independently represent 0 or 1 and satisfy $0 \leq x + y \leq 1$.

The arylene group represented by each of $Ar^6$, $Ar^7$, $Ar^8$ and $Ar^9$ is the remaining atomic group provided by removing two hydrogen atoms from an aromatic hydrocarbon. Examples of the aromatic hydrocarbon include a compound having a condensed ring and a compound having at least two independent benzene rings or condensed rings directly bonded or bonded via e.g., a vinylene group.

A divalent heterocyclic group represented by each of $Ar^6$, $Ar^7$, $Ar^8$ and $Ar^9$ is the remaining atomic group provided by removing two hydrogen atoms from a heterocyclic compound. The number of carbon atoms of the divalent heterocyclic group is usually 4 to 60. The heterocyclic compound refers to an organic compound having a cyclic structure and containing not only carbon atoms but also hetero atoms such as oxygen, sulfur, nitrogen, phosphorus, boron as elements constituting the ring. As the divalent heterocyclic group, a divalent aromatic heterocyclic group is preferable.

An aryl group represented by each of $Ar^{10}$, $Ar^{11}$ and $Ar^{12}$ is the remaining atomic group provided by removing a single hydrogen atom from an aromatic hydrocarbon. The aromatic hydrocarbon is as defined above.

A monovalent heterocyclic group represented by each $Ar^{10}$, $Ar^{11}$ and $Ar^{12}$ refers to as the remaining atomic group provided by removing a single hydrogen atom from a heterocyclic compound. The number of carbon atoms of the monovalent heterocyclic group is usually 4 to 60. The heterocyclic compound is as defined above. As the monovalent heterocyclic group, a monovalent aromatic heterocyclic group is preferable.

When the compound having a residue(s) of a nitrogen-containing compound and a residue(s) of a nitrogen-containing polycyclic compound is a polymer compound, the polystyrene equivalent weight average molecular weight of the compound is preferably $3 \times 10^2$ or more in view of film formation property, more preferably, $3 \times 10^2$ to $1 \times 10^7$, further preferably, $1 \times 10^3$ to $1 \times 10^7$, and particularly preferably, $1 \times 10^4$ to $1 \times 10^7$.

The compound having a residue(s) of a nitrogen-containing compound and a residue(s) of a nitrogen-containing polycyclic compound can be used in a wide emission wavelength region. The $T_1$ energy value of the compound is preferably 3.0 eV or more, more preferably 3.2 eV or more, further preferably 3.4 eV or more, and particularly preferably, 3.5 eV or more. Furthermore, the upper limit is usually 5.0 eV

The absolute value of the HOMO energy level of the compound having a residue(s) of a nitrogen-containing compound and a residue(s) of a nitrogen-containing polycyclic compound is preferably 6.2 eV or less, more preferably, 5.9 eV or less, and further preferably 5.6 eV or less. Furthermore, the lower limit is usually 5.0 eV

The absolute value of the LUMO energy level of the compound having a residue(s) of a nitrogen-containing compound and a residue(s) of a nitrogen-containing polycyclic compound is preferably 1.5 eV or more, more preferably, 1.7 eV or more, further preferably 1.9 eV or more, especially preferably 2.0 eV or more, and particularly preferably 2.2 eV or more. Furthermore, the upper limit is usually 4.0 eV.

In the specification, a $T_1$ energy value of each compound and a value of an LUMO energy level are the values calculated by a computational scientific approach. In the specification, as the computational scientific approach, optimization of a ground state structure was performed by the Hartree-Fock (HF) method using a quantum chemical calculation program, Gaussian03, and then, in the optimized structure, a $T_1$ energy value and a value of an LUMO energy level were obtained by using a B3P86 level time-dependent density functional method. At this time, as a basis function, 6-31g* was used. When 6-31g* cannot be used as a basis function, LANL2DZ is used. In the present invention, the absolute value of the "value of an LUMO energy level" (more specifically, in the case where an LUMO energy level is expressed by a negative value, the absolute value refers to the value provided by eliminating the negative symbol from the negative value) is important.

In the case where the compound having a residue(s) of a nitrogen-containing compound and a residue(s) of a nitrogen-containing polycyclic compound is constituted of single-type repeating units, assuming that the unit is represented by A, the compound having a residue(s) of a nitrogen-containing compound and a residue(s) of a nitrogen-containing polycyclic compound is expressed by the following formula:

$$\left( A \right)_n$$

wherein n represents the number of polymerization units. Herein, a $T_1$ energy value and a value of an LUMO energy level are calculated in the cases of structures given by n = 1, 2 and 3. The $T_1$ energy value and the value of an LUMO energy level calculated are linearly approximated as a function of (1/n). The values of n = ∞ of this case are defined as the $T_1$ energy value and the value of the LUMO energy level of the polymer compound.

In the case where there is more than one repeating unit for constituting the compound having a residue(s) of a nitrogen-containing compound and a residue(s) of a nitrogen-containing polycyclic compound, $T_1$ energy values for all cases assuming that n = ∞ (wherein n is the number of repeating units polymerized) are calculated in the same manner as above. Of them, the lowest $T_1$ energy value is defined as the $T_1$ energy value of the compound. The value of the LUMO energy level of the polymer compound is defined as a value at n = ∞ in the repeating unit providing the lowest $T_1$ energy value.

The compound having a residue(s) of a nitrogen-containing compound and a residue(s) of a nitrogen-containing polycyclic compound preferably has a heterocyclic structure constituting the nitrogen-containing compound and the nitrogen-containing polycyclic compound and a partial structure adjacent to the heterocyclic structure (where the partial structure has at least two π conjugated electrons). The dihedral angle between the heterocyclic structure and the partial structure adjacent to the heterocyclic structure is preferably 40° or more, more preferably 55° or more, further preferably 70° or more, and particularly preferably 80° or more.

Furthermore, in the compound having a residue(s) of a nitrogen-containing compound and a residue(s) of a nitrogen-containing polycyclic compound, dihedral angles between aromatic rings and hetero aromatic rings including the heterocyclic structure all are preferably 40° or more, more preferably 55° or more, further preferably 70° or more, and particularly preferably 80° or more. Furthermore, to obtain such a dihedral angle, it is preferable to have a partial structure represented by the above formula (A-3).

Furthermore, in the specification, the dihedral angle refers to an angle calculated from the optimized structure in a ground state. The dihedral angle is defined, for example, by a carbon atom ($a_1$) which is located at a bonding position and the carbon atom or nitrogen atom ($a_2$) located next to $a_1$ in the compound having a residue(s) of a nitrogen-containing compound and a residue(s) of a nitrogen-containing polycyclic compound, and an atom ($a_3$) located in the bonding position and an atom ($a_4$) located next to $a_3$ in a structure bonding to the heterocyclic structure. If more than one atom ($a_2$) or atom ($a_4$) can be selected herein, dihedral angles of all cases are calculated. Of them, the lowest value (90° or less) is employed as the dihedral angle. The atoms ($a_3$) and ($a_4$) are atoms having π-conjugated electrons, and more preferably, are carbon atoms, nitrogen atoms, silicon atoms and phosphorus atoms. In the specification, calculation is made from an optimized structure (more specifically, the structure produced with the lowest production energy) at n = 3 (n is the number of polymerization units) in a ground state obtained by a computational scientific approach. In the compound having a heterocyclic structure, there is more than one dihedral angle. In this case, all dihedral angles of the compound preferably satisfy the above conditions.

As the compound having a residue(s) of a nitrogen-containing compound and a residue(s) of a nitrogen-containing polycyclic compound, compounds represented by the following formulas are mentioned. In the formulas, R* represents a hydrogen atom or a substituent. Examples of the substituent represented by R* include a halogen atom, an alkyl group, an alkoxy group, an alkylthio group, an aryl group that may have a substituent, an aryloxy group, an arylthio group, an arylalkyl group, an arylalkyloxy group, an arylalkylthio group, an acyl group, an acyloxy group, an amide group, an acid imide group, an imide residue, a substituted amino group, a substituted silyl group, a substituted silyloxy group, a substituted silylthio group, a substituted silylamino group, a monovalent heterocyclic group that may have a substituent, an heteroaryl group that may have a substituent, a heteroaryloxy group, a heteroarylthio group, an arylalkenyl group, an arylethynyl group, a substituted carboxyl group and a cyano group. More than one R* may be the same or different. As $R^*$, an alkyl group, an alkoxy group, an aryl group that may have a substituent and a heteroaryl group that may have a substituent are more preferable. More than one R* may be the same or different.

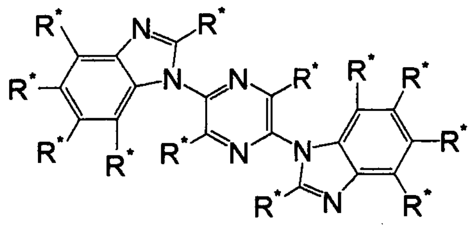
(3-1)

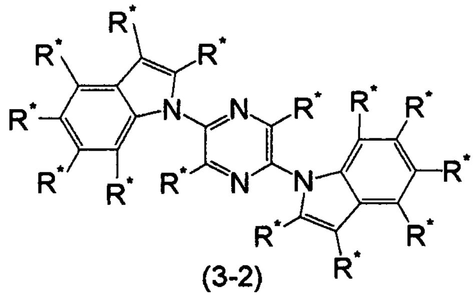
(3-2)

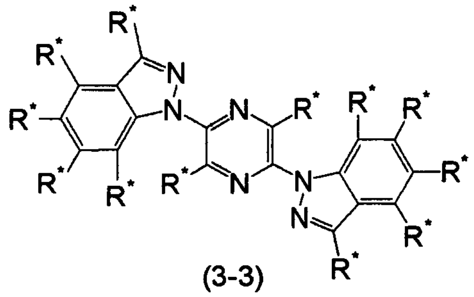
(3-3)

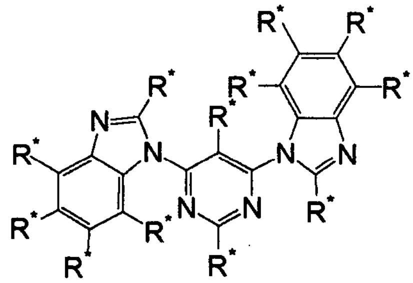
(3-4)

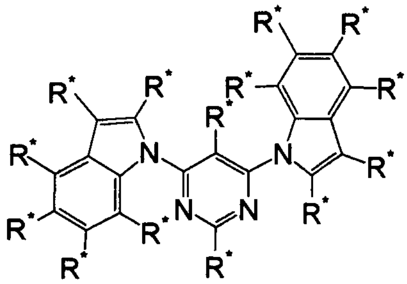
(3-5)

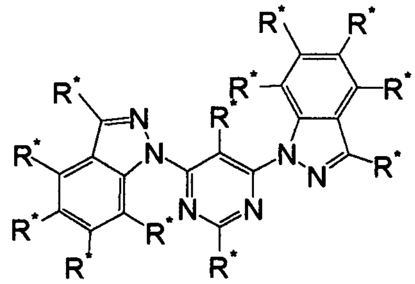
(3-6)

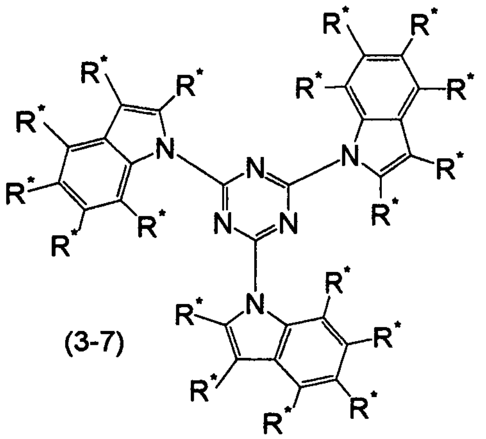
(3-7)

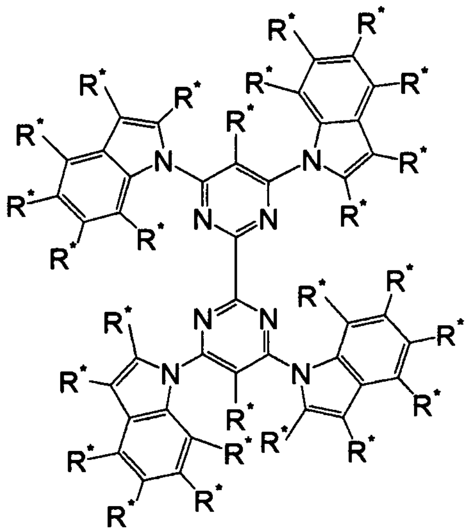
(3-8)

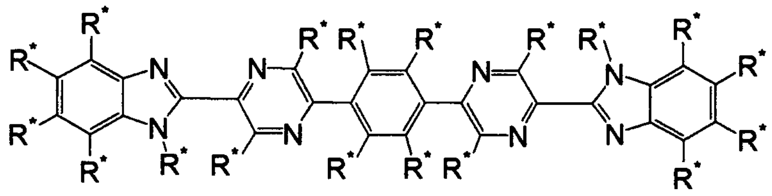

(3-9)

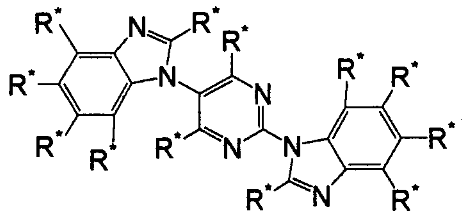

(3-10)

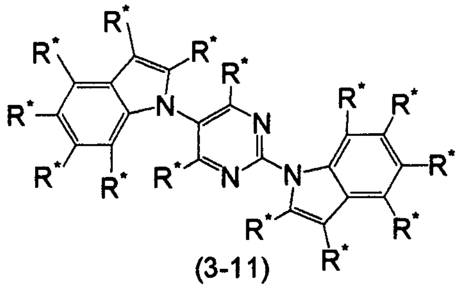

(3-11)

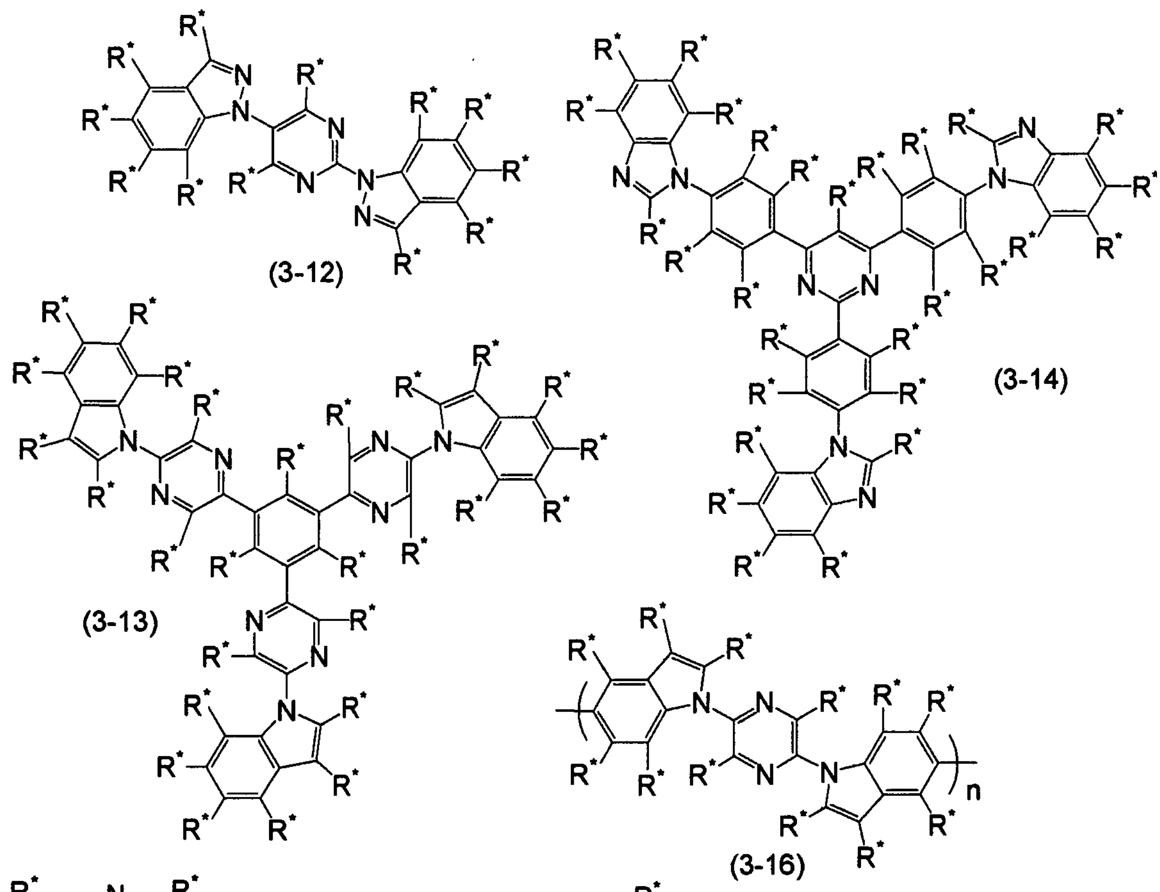

(3-12)

(3-14)

(3-13)

(3-16)

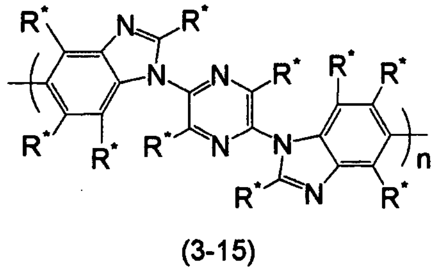

(3-15)

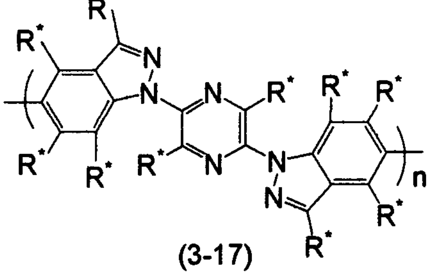

(3-17)

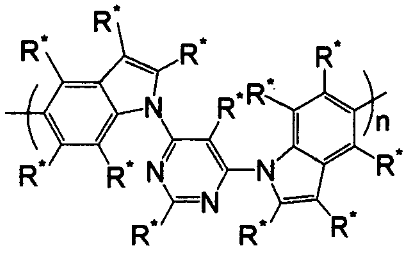

(3-18)

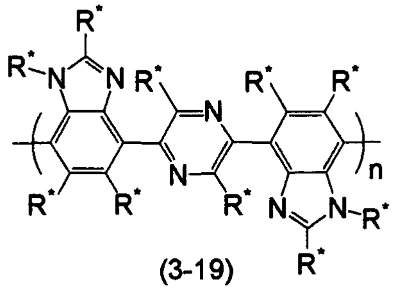

(3-19)

wherein n represents the number of polymerization units.

Furthermore, as the compound having a residue(s) of a nitrogen-containing compound and a residue(s) of a nitrogen-containing polycyclic compound, the following compounds may also be mentioned.

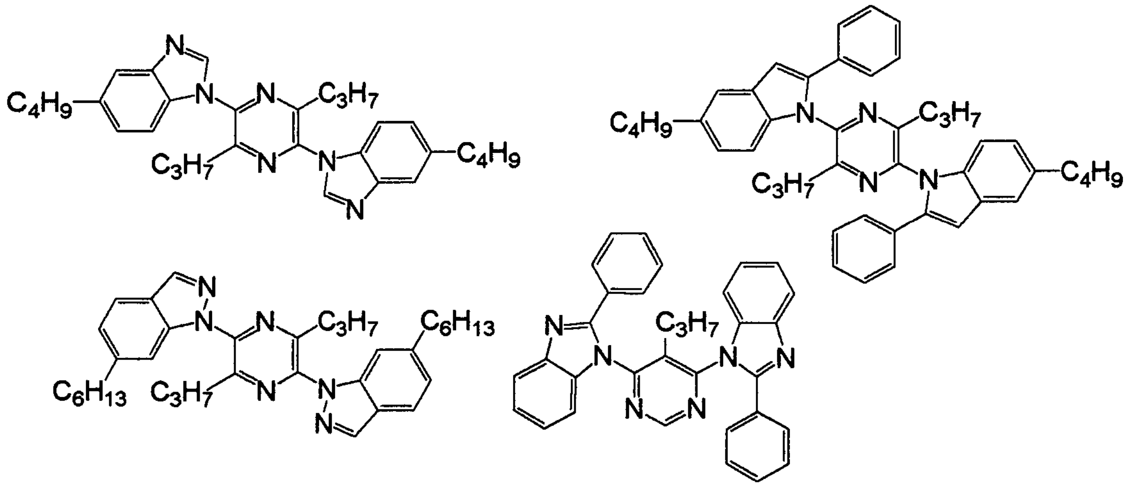

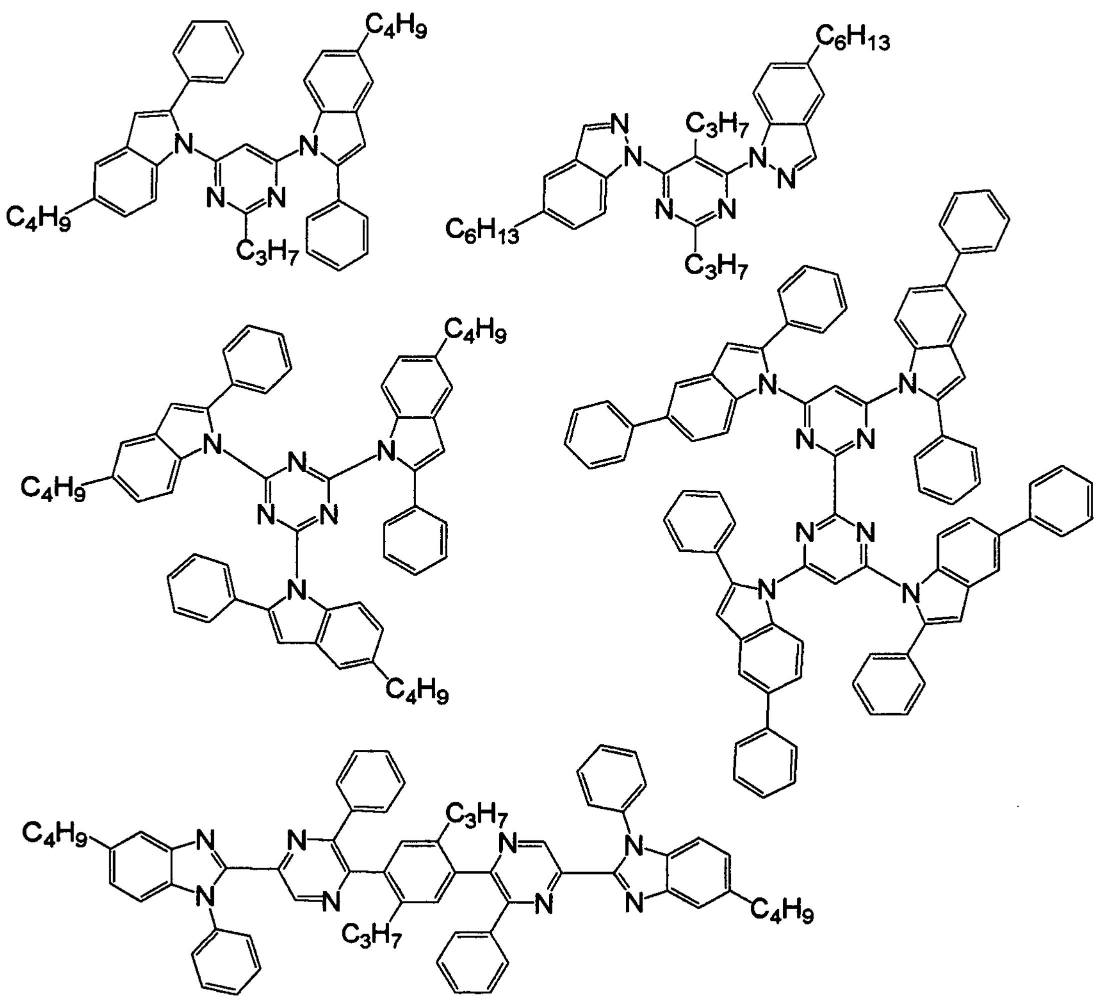

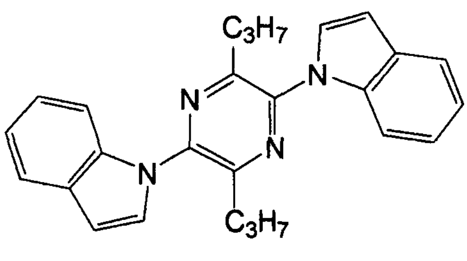

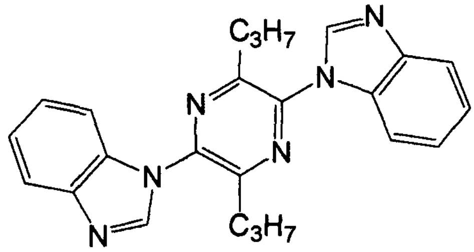

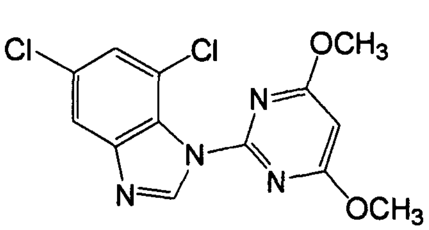

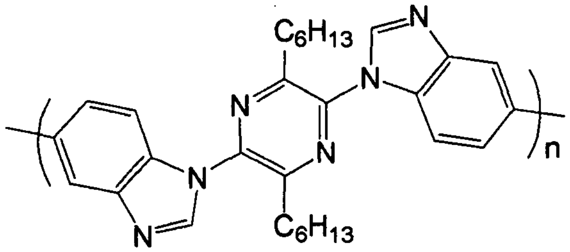

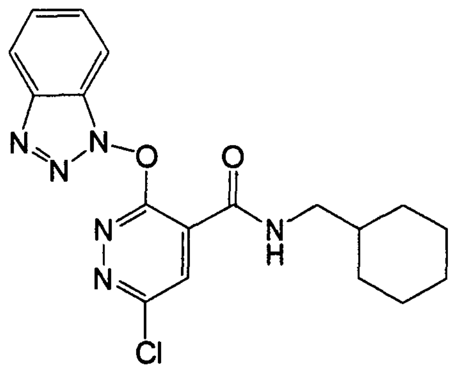

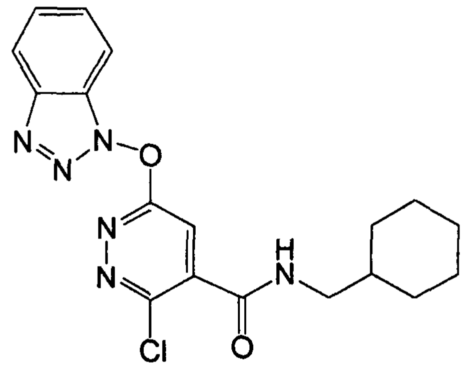

As the phosphorescent compound, triplet emission complexes and compounds that have been used as a lower-molecular EL emitting material are mentioned. These are disclosed, for example, in Nature, (1998), 395, 151, Appl. Phys. Lett. (1999), 75(1), 4, Proc. SPIE-Int. Soc. Opt. Eng. (2001), 4105 (Organic Light-Emitting Materials and DevicesIV), 119, J. Am. Chem. Soc., (2001), 123, 4304, Appl. Phys. Lett., (1997), 71(18), 2596, Syn. Met., (1998), 94(1), 103, Syn. Met., (1999), 99(2), 1361, Adv. Mater., (1999), 11(10), 852, Inorg. Chem., (2003), 42, 8609, Inorg. Chem., (2004), 43, 6513, Journal of the SID 11/1, 161 (2003), WO2002/066552 WO2004/020504, and WO2004/020448. Of these, the total of a square of an orbital coefficient of the outermost shell d-orbital of the central metal in the HOMO of a metal complex preferably occupies not less than 1/3 ratio of the total of a square of orbital coefficients of all atoms in order to obtain a high luminous efficiency. For example, ortho-metalated complexes, which is a transition metal having a central metal belonging to the 6th period, are mentioned.

The central metal of the triplet emission complex, which is usually a metal atom of an atomic number of 50 or more, having a spin-orbit interaction and capable of causing the intersystem crossing between a singlet state and a triplet state, include preferably atoms such as gold, platinum, iridium, osmium, rhenium, tungsten, europium, terbium, thulium, dysprosium, samarium, praseodymium, gadolinium and ytterbium; more preferably atoms such as gold, platinum, iridium, osmium, rhenium and tungsten; further preferably atoms such as gold, platinum, iridium, osmium and rhenium; particularly preferably atoms such as gold, platinum, iridium and rhenium, and especially preferably atoms such as platinum and iridium.

Examples of the ligand of the triplet emission complex include 8-quinolinol and a derivative thereof, benzoquinolinol and a derivative thereof, and 2-phenyl-pyridine and a derivative thereof.

As the phosphorescent compound, in view of solubility, a compound having a substituent such as an alkyl group, an alkoxy group, an aryl group that may have a substituent and a heteroaryl group that may have a substituent are preferable. Furthermore, the substituent preferably has 3 or more atoms in total, except a hydrogen atom, more preferably 5 or more, further preferably 7 or more, and particularly preferably 10 or more. Furthermore, at least one of the substituents is preferably present in each ligand. The types of substituents may be the same or different per ligand.

As the phosphorescent compound, the following compounds are mentioned.

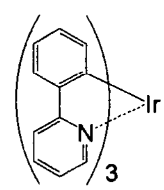

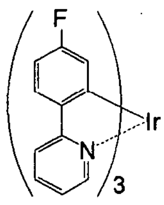

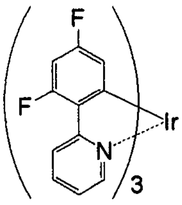

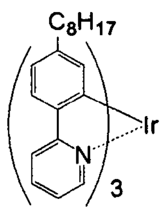

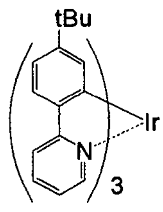

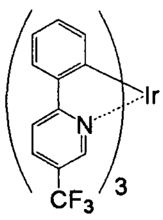
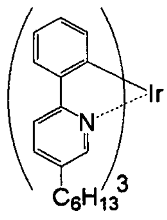
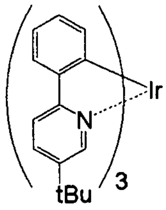
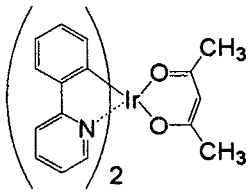

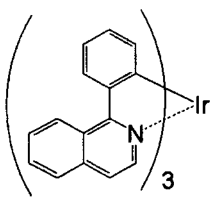
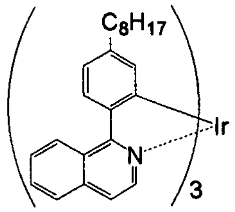
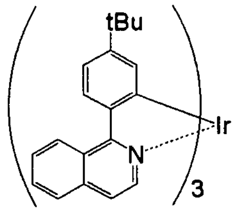
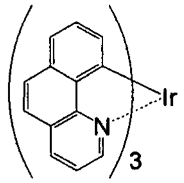

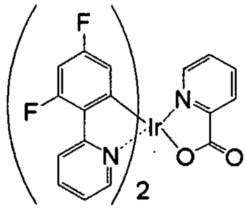
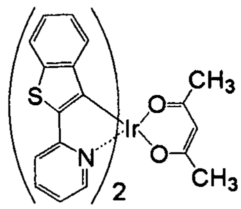
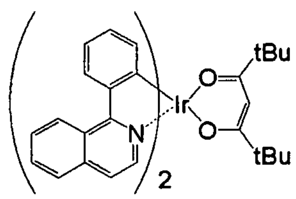

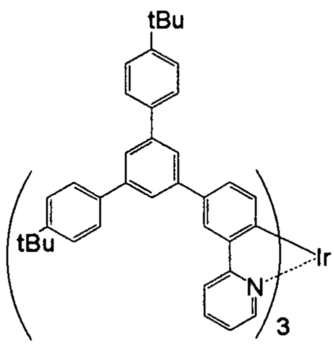
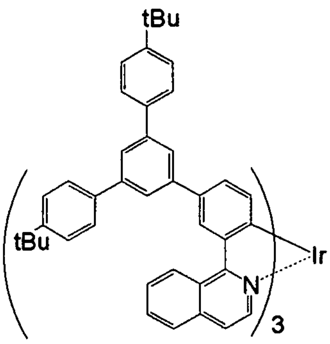

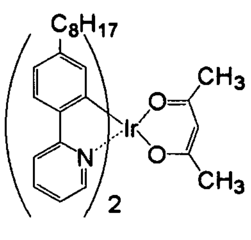
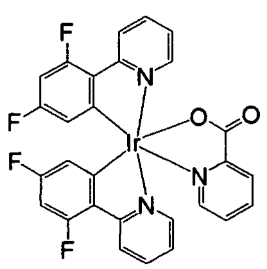

The amount of phosphorescent compound in the composition of the present invention varies depending upon the type of organic compound to be used in combination and the properties to be optimized; however, the amount is

usually, 0.01 to 80 parts by weight, based on 100 parts by weight of the compound having a residue(s) of a nitrogen-containing compound and a residue(s) of a nitrogen-containing polycyclic compound, preferably, 0.1 to 30 parts by weight, more preferably, 0.1 to 15 parts by weight, and particularly preferably, 0.1 to 10 parts by weight. Note that in the composition of the present invention, the compound having a residue(s) of a nitrogen-containing compound and a residue (s) of a nitrogen-containing polycyclic compound and the phosphorescent compound may each be used alone or in combination of two or more thereof

The composition of the present invention may contain an optional component other than the compound having a residue(s) of a nitrogen-containing compound and a residue(s) of a nitrogen-containing polycyclic compound and the phosphorescent compound as long as the object of the invention is not damaged. As the optional component, for example, a hole transport material, an electron transport material and an antioxidant are mentioned.

Examples of the hole transport material include well-known hole transport materials for an organic EL device, such as an aromatic amine, a carbazole derivative and a polyparaphenylene derivative.

Examples of the electron transport material include well-known electron transport materials for an organic EL device, such as metal complexes of an oxadiazole derivative, anthraquinodimethane and a derivative thereof, benzoquinone and a derivative thereof, naphthoquinone and a derivative thereof, anthraquinone and a derivative thereof, tetracyanoanthraquinodimethane and a derivative thereof, a fluorenone derivative, diphenyldicyanoethylene and a derivative thereof, a diphenoquinone derivative, and 8-hydroxyquinoline and a derivative thereof.

In the composition of the present invention, the $T_1$ energy value (ETH) of the compound having a residue(s) of a nitrogen-containing compound and a residue(s) of a nitrogen-containing polycyclic compound and the $T_1$ energy value (ETG) of the phosphorescent compound preferably satisfy the following expression:

$$\mathrm{ETH} > \mathrm{ETG} \quad (\mathrm{eV})$$

in view of highly efficient light emission, more preferably satisfy

$$\mathrm{ETH} > \mathrm{ETG}+0.1\ (\mathrm{eV})$$

and further preferably

$$\mathrm{ETH} > \mathrm{ETG}+0.2\ (\mathrm{eV}).$$

The film of the present invention can be manufactured by using the composition, etc. of the present invention. For preparing the film, solution coating, vapor deposition and transfer, etc. can be used. As the solution coating, a spin coating method, a casting method, a microgravure coating method, a gravure coating method, a bar coating method, a roll coating method, a wire-bar coating method, dip coating method, a spray coating method, a screen printing method, a flexo printing method, an off-set printing method and an inkjet printing method etc. may be used.

As the solvent, a solvent capable of dissolving or uniformly dispersing the composition is preferable. Examples of the solvent include chlorine solvents (chloroform, methylene chloride, 1,2-dichloroethane, 1,1,2-trichloroethane, chlorobenzene, o-dichlorobenzene, etc.), ether solvents (tetrahydrofuran, dioxane, etc.), aromatic hydrocarbon solvents (toluene, xylene, etc.), aliphatic hydrocarbon solvents (cyclohexane, methylcyclohexane, n-pentane, n-hexane, n-heptane, n-octane, n-nonane, n-decane, etc.), ketone solvents (acetone, methyl ethyl ketone, cyclohexanone, etc.), ester solvents (ethyl acetate, butyl acetate, ethyl cellosolve acetate, etc.), polyhydric alcohols and derivatives thereof (ethylene glycol, ethylene glycol monobutyl ether, ethylene glycol monoethyl ether, ethylene glycol monomethyl ether, dimethoxyethane, propylene glycol, diethoxymethane, triethylene glycol monoethyl ether, glycerin, 1,2-hexanediol, etc.), alcohol solvents (methanol, ethanol, propanol, isopropanol, cyclohexanol, etc.), sulfoxide solvents (dimethylsulfoxide, etc.) and amide solvents (N-methyl-2-pyrrolidone, N,N-dimethylformamide, etc.). A solvent can be selected from these and put in use. Furthermore, these organic solvents may be used alone or in combination of two or more thereof.

When the inkjet printing method is used, to improve ejection property from a head and uniformity, etc., a solvent in a solution and additives can be selected according to known methods. In this case, the viscosity of the solution is preferably 1 to 100 mPa·s at 25°C. Furthermore, if vaporization is significant, it tends to be difficult to repeat ejection from a head. In view of these, examples of a preferable solvent include a single solvent or solvent mixture containing anisole, bicyclohexyl, xylene, tetralin and dodecyl benzene. Generally, a solution for inkjet printing suitable for a composition to be used can be obtained by a method of mixing more than one solvent, a method of controlling the concentration thereof in a solution of a composition and the like.

<Polymer compound>

The polymer compound of the present invention is a polymer compound containing: a residue(s) of at least one nitrogen-containing compound selected from the group consisting of nitrogen-containing compounds represented by the above formulas (1-1), (1-2), (1-3) and (1-4); a residue(s) of at least one nitrogen-containing polycyclic compound selected from the group consisting of nitrogen-containing polycyclic compounds represented by the above formulas (2-1), (2-2), (2-3) and (2-4); and a residue(s) of a phosphorescent compound. The phosphorescent compound, the nitrogen-containing compound and the nitrogen-containing polycyclic compound are the same exemplified in the above section of composition. Examples of the polymer compound of the present invention include (1) a polymer compound having a residue(s) of a phosphorescent compound in the main chain, (2) a polymer compound having a residue(s) of a phosphorescent compound at an end, and (3) a polymer compound having a residue(s) of a phosphorescent compound at a side chain.

<Light-emitting device>

Next, the light-emitting device of the present invention will be described.
The light-emitting device of the present invention is prepared by using the composition, etc. of the present invention. Usually, the composition, etc. of the present invention are contained in at least a part of the layer provided between electrodes consisting of an anode and a cathode. They are preferably contained as a light-emitting layer in the form of the light-emitting film. Furthermore, in view of improving performance such as luminous efficiency and durability, a known layer having another function may be contained. Examples of such a layer include a charge transport layer (more specifically, hole transport layer, electron transport layer), a charge block layer (more specifically, hole block layer, electron block layer), a charge injection layer (more specifically, hole injection layer, electron injection layer), and a buffer layer. Note that in the light-emitting device of the present invention, the light-emitting layer, charge transport layer, charge block layer, charge injection layer and buffer layer, etc. each may be formed of a single layer or two or more layers.
The light-emitting layer is a layer having a function of emitting light. The hole transport layer is a layer having a function of transporting holes. The electron transport layer is a layer having a function of transporting electrons. The electron transport layer and the hole transport layer are collectively referred to as a charge transport layer. Furthermore, the charge block layer is a layer having a function of confining holes or electrons in the light-emitting layer. The layer for transporting electrons and confining holes is referred to as a hole block layer and a layer for transporting holes and confining electrons is referred to as an electron block layer.
As the buffer layer, a layer provided in adjacent to an anode and containing a conductive polymer compound is mentioned.
As the light-emitting device of the present invention, the following structures a) to q) are mentioned.

a) Anode/light-emitting layer/cathode
b) Anode/hole transport layer/light-emitting layer/cathode
c) Anode/light-emitting layer/electron transport layer/cathode
d) Anode/light-emitting layer /hole block layer/cathode
e) Anode/hole transport layer/light-emitting layer/electron transport layer/cathode
f) Anode/charge injection layer/light-emitting layer/cathode
g) Anode/light-emitting layer/charge injection layer/cathode
h) Anode/charge injection layer/light-emitting layer/charge injection layer/cathode
i) Anode/charge injection layer/hole transport layer/light-emitting layer/cathode
j) Anode/hole transport layer/light-emitting layer/charge injection layer/cathode
k) Anode/charge injection layer/hole transport layer/light-emitting layer/charge injection layer/cathode
l) Anode/charge injection layer/light-emitting layer/electron transport layer/cathode
m) Anode/light-emitting layer/electron transport layer/charge injection layer/cathode
n) Anode/charge injection layer/light-emitting layer/electron transport layer/charge injection layer/cathode
o) Anode/charge injection layer/hole transport layer/light-emitting layer/electron transport layer/cathode
p) Anode/hole transport layer/light-emitting layer/electron transport layer/charge injection layer/cathode
q) Anode/charge injection layer/hole transport layer/light-emitting layer/electron transport layer/charge injection layer/cathode (herein, the symbol "/" means that layers are laminated next to each other. The same applies hereinafter. Note that the light-emitting layer, hole transport layer and electron transport layer may each independently be formed of two or more thereof).

In the case where the light-emitting device of the present invention has a hole transport layer (usually, the hole transport layer contains a hole transport material), known materials are mentioned as the hole transport material. Examples thereof include polymer hole transport materials such as polyvinylcarbazole and a derivative thereof, polysilane and a derivative thereof, polysiloxane derivative having an aromatic amine in a side chain or the main chain, a pyrazoline derivative, an arylamine derivative, a stilbene derivative, a triphenyldiamine derivative, polyaniline and a derivative thereof, polythiophene and a derivative thereof, polypyrrole and a derivative thereof, poly(p-phenylenevinylene) and a derivative thereof, and poly(2,5-thienylenevinylene) and a derivative thereof; and further include the compounds described in JP 63-70257 A, JP 63-175860 A, JP 2-135359 A, JP 2-135361 A, JP 2-209988A, JP 3-37992 A and JP 3-152184 A.

In the case where the light-emitting device of the present invention has an electron transport layer (usually, the electron transport layer contains an electron transport material), known materials are mentioned as the electron transport material. Examples thereof include an oxadiazole derivative, anthraquinodimethane and a derivative thereof, benzoquinone and a derivative thereof, naphthoquinone and a derivative thereof, anthraquinone and a derivative thereof, tetracyanoanthraquinodimethane and a derivative thereof, a fluorenone derivative, diphenyldicyanoethylene and a derivative thereof, a diphenoquinone derivative, 8-hydroxyquinoline and a complex of a derivative thereof, polyquinoline and a derivative thereof, polyquinoxaline and a derivative thereof, and polyfluorene and a derivative thereof.

The film thicknesses of the hole transport layer and electron transport layer, whose optimum values thereof vary depending upon the material to be used, may be appropriately selected so as to obtain an appropriate driving voltage and luminous efficiency; however, the thickness is required to be sufficiently thick such that at least pin holes are not formed. If the film is extremely thick, the driving voltage of the device becomes high and thus not preferable. Therefore, the film thicknesses of the hole transport layer and electron transport layer are for example, 1 nm to 1 $\mu$m, preferably 2 nm to 500 nm, and further preferably 5 nm to 200 nm.

Furthermore, of the charge transport layers provided in adjacent to an electrode, a charge transport layer having a function of improving a charge injection efficiency from the electrode and an effect of reducing the driving voltage of the device, is sometimes called particularly as a charge injection layer (that is, a general name of a hole injection layer, and an electron injection layer. The same applies hereinafter).

Furthermore, to improve adhesion with an electrode and improve charge injection form an electrode, the charge injection layer or an insulating layer may be provided in adjacent to the electrode (usually, having an average thickness of 0.5 nm to 4 nm). Furthermore, to improve the adhesion of the interface and prevent contamination, etc., a thin buffer layer may be inserted into the interface of a charge transport layer and a light-emitting layer.

The lamination order of the layers and number of layers and the thickness of individual layers can be appropriately selected in consideration of luminous efficiency and the life of the device.

Examples of the charge injection layer include a layer containing a conductive polymer compound, a layer provided between an anode and a hole transport layer and having an intermediate ionization potential between an anode material and a hole transport material contained in the hole transport layer, and a layer provided between a cathode and an electron transport layer and having an intermediate electron affinity value between a cathode material and an electron transport material contained in the hole transport layer.

The material to be used in the charge injection layer may be appropriately selected in consideration of the materials of electrodes and adjacent layers. Examples thereof include polyaniline and a derivative thereof, polythiophene and a derivative thereof, polypyrrole and a derivative thereof, polyphenylenevinylene and a derivative thereof, polythienylenevinylene and a derivative thereof, polyquinoline and a derivative thereof, polyquinoxaline and a derivative thereof, a conductive polymer compound such as a polymer containing an aromatic amine structure in the main chain or a side chain, a metal phthalocyanine (copper phthalocyanine, etc.) and carbon.

The insulating layer has a function of facilitating charge injection. Examples of the material for the insulating layer include a metal fluoride, a metal oxide and an organic insulating material. As the light-emitting device having the insulating layer provided therein, for example, a light-emitting device having an insulating layer provided in adjacent to a cathode and a light-emitting device having an insulating layer provided in adjacent to an anode are mentioned.

The light-emitting device of the present invention is usually formed on a substrate. Any substrate may be used as long as it does not change even if an electrode is formed thereon and an organic material layer is formed thereon. Examples thereof include glass, plastic, a polymer film and silicon. In the case of an opaque substrate, an opposite electrode is preferably transparent or semitransparent.

At least one of the anode and the cathode present in the light-emitting device of the present invention is usually transparent or semitransparent. Of them, the anode side is preferably transparent or semitransparent.

As a material for an anode, usually a conductive metal oxide film and a semitransparent metal film, etc. are used. Specific examples thereof include films (NESA, etc.) prepared by using conductive inorganic compounds such as indium oxide, zinc oxide, tin oxide, and a complex thereof, namely, indium tin oxide (ITO), indium zinc oxide; gold, platinum, silver and copper. ITO, indium zinc oxide, and tin oxide are preferable. As the preparation method, a vacuum vapor deposition method, a sputtering method, an ion plating method and a plating method, etc. are mentioned. Furthermore, as the anode, an organic transparent conductive film of polyaniline or a derivative thereof, and polythiophene or a derivative thereof etc. may be used. Note that the anode may be formed of a laminate structure of 2 layers or more.

As a material for a cathode, usually, a material having a small work function is preferable. Examples thereof include metals such as lithium, sodium, potassium, rubidium, cesium, beryllium, magnesium, calcium, strontium, barium, aluminum, scandium, vanadium, zinc, yttrium, indium, cerium, samarium, europium, terbium, ytterbium, and alloys formed from at least two of metals selected from them or alloys of at least one of metals selected from them and at least one of gold, silver, platinum, copper, manganese, titanium, cobalt, nickel, tungsten and tin, graphite or a graphite intercalation compound. Examples of the alloy include a magnesium-silver alloy, a magnesium-indium alloy, a magnesium-aluminum alloy, an indium-silver alloy, a lithium-aluminum alloy, a lithium-magnesium alloy, a lithium-indium alloy, a calcium-aluminum alloy. Note that the cathode may be formed of a laminate structure of 2 layers or more.

The light-emitting device of the present invention can be used, for example, as a planar light source, a display (for example, a segment display, a dot matrix display, a liquid crystal display) and backlights thereof (for example, a liquid crystal display having the light-emitting device as a backlight).

To obtain planer emission of light using the light-emitting device of the present invention, a planar anode and cathode are arranged so as to overlap them. Furthermore, to obtain patterned emission of light, there are a method of placing a mask having a patterned window on the surface of the planar light-emitting device, a method of forming an extremely thick organic material layer in a non light-emitting section such that light is not substantially emitted, and a method of forming a patterned electrode as either one of an anode and cathode or both electrodes. Patterns are formed by any one of these methods and electrodes are arranged so as to independently turn ON/OFF. In this manner, a segment-type display device capable of displaying numeric characters and letters, and simple symbols, etc. can be obtained. Furthermore, to obtain a dot matrix device, an anode and a cathode are formed in the form of stripe and arranged so as to cross perpendicularly. A partial color display and multi color display can be provided by a method of distinctively applying more than one light-emitting material different in luminous color and a method of using a color filter or a fluorescence conversion filter. A dot-matrix device can be passively driven or may be actively driven in combination with TFT, etc. These display devices can be used as displays for computers, televisions, mobile terminals, mobile phones, car-navigation and view finders of video cameras, etc.

Furthermore, the planar light-emitting device is usually an autonomous light-emitting thin device and can be preferably used as a planar light source for a backlight of a liquid crystal display and light (for example, planar light, a light source for planar light), etc. Furthermore, if a flexible substrate is used, the light-emitting device can be used as a curved-surface light source, light and a display, etc.

The composition, etc. of the present invention can be also used as a semiconductor material such as an organic semiconductor material, a light-emitting material, an optical material and a conductive material (for example, applied by doping). Furthermore, films such as light-emitting film, a conductive film and an organic semiconductor film can be prepared by using the composition, etc. of the present invention.

The composition, etc. of the present invention can be used to form a conductive film and a semiconductor film in the same manner as in a preparation method for a light-emitting film to be used in the light emitting layer of the light-emitting device, and formed into a device. In the semiconductor film, a larger value of an electron mobility or hole mobility is preferably not less than $10^{-5}$ $cm^2$/V/second. Furthermore, an organic semiconductor film can be used in organic solar batteries and organic transistors, etc.

EXAMPLES

Hereinafter, Examples will be described to explain the present invention more specifically; however, the present invention is not limited to these.

<Example 1>

A compound (C-1) represented by the following formula:

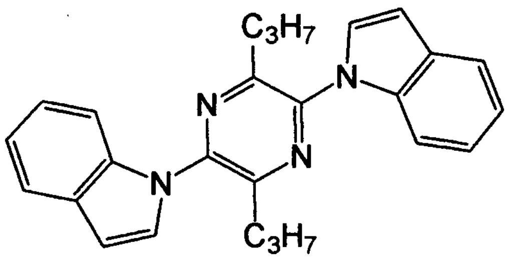

(C-1)

had a $T_1$ energy value of 3.1 eV, an absolute value of an LUMO energy level ($E_{LUMO}$) of 2.2 eV, an absolute value of an HOMO energy level ($E_{HOMO}$) of 6.2 eV and the smallest dihedral angle of 61°.
Calculation of parameters was performed by using the structure of the compound (C-1). To describe more specifically, the structure of the compound (C-1) was optimized by an HF method. At this time, 6-31G* was used as a basis function. Thereafter, a value of an LUMO energy level, a value of an HOMO energy level and a $T_1$ energy value were calculated by a time-dependent density functional method of B3P86 level using the same basis function. Furthermore, dihedral angles were calculated from an optimized structure. Of the more than one dihedral angle present herein, the smallest dihedral angle alone is entered.
It can be confirmed that a light-emitting device, which is prepared by using a composition containing the compound (C-1) and a phosphorescent compound, is excellent in luminous efficiency.

<Example 2>

A compound (C-2) represented by the following formula:

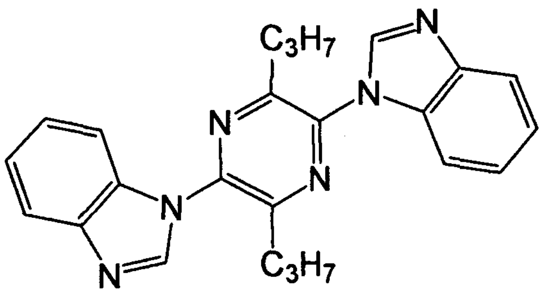

(C-2)

had a $T_1$ energy value of 3.4 eV, an absolute value of an LUMO energy level ($E_{LUMO}$) of 2.5 eV, and the smallest dihedral angle of 59°. Parameters were calculated by the computational scientific approach in the same manner as in Example 1.
It can be confirmed that a light-emitting device, which is prepared by using a composition containing a compound (C-2) and a phosphorescent compound, is excellent in luminous efficiency.

<Example 3>

A polymer compound (P-1) represented by the following formula:

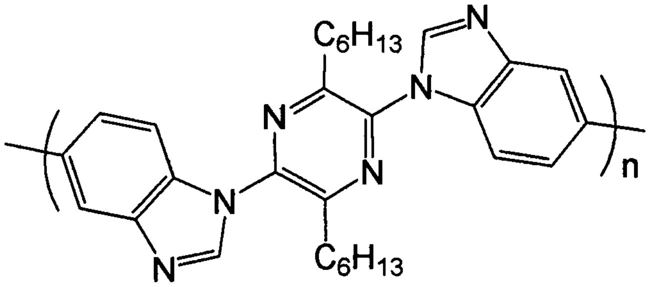

(P-1)

wherein n is the number of polymerization units,
had a $T_1$ energy value of 3.2 eV, and an absolute value of an LUMO energy level ($E_{LUMO}$) of 2.6 eV, which were extrapolation values at n = ∞, and the smallest dihedral angle of 46°.
Parameters were calculated by the aforementioned computational scientific approach. To describe more specifically, the repeating unit (M-1) represented by the following formula (M-1) in a polymer compound (P-1) was simplified as shown in the following formula (M-1a) and subjected to calculation. The adequacy of simplifying the chemical structure was confirmed by the method described in JP 2005-126686 A based on the fact that the dependency of the $T_1$ energy value and the value of an LUMO energy level upon of the length of an alkyl side chain is low. The simplified repeating unit (M-1a) was used to optimize the structure by the HF method when n = 1, 2 and 3.

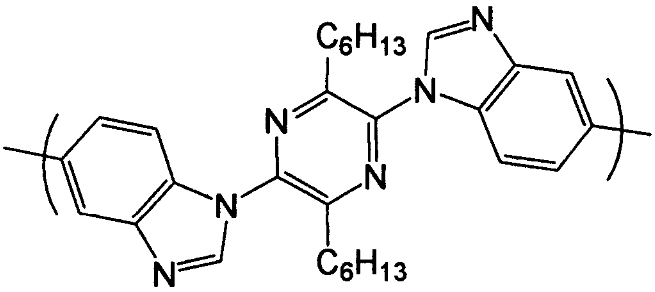

(M-1)

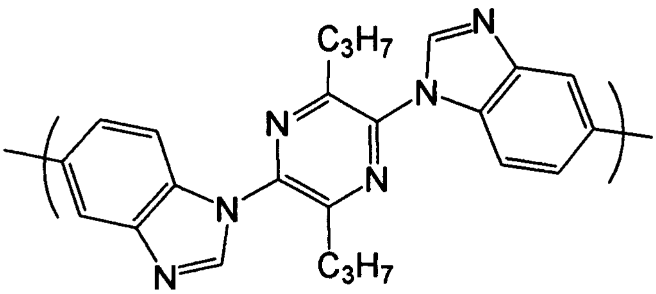

(M-1a)

At this time, as the basis function, 6-31G* was used. Thereafter, a value of an LUMO energy level and a T1 energy value were calculated by using the same basis function and according to the time-dependent density functional method of B3P86 level. A value of an LUMO energy level calculated in each n and T1 energy value was expressed as an inverse function (1/n) of n and the extrapolation value at n = ∞ was a value of this function at 1/n = 0. Furthermore, dihedral angles were calculated from an optimized structure at n = 3 (n is the number of polymerization units). Of the more than one dihedral angle, the smallest value alone was entered.
It can be confirmed that a light-emitting device prepared by using a composition containing a polymer compound (P-1) and a phosphorescent compound, is excellent in luminous efficiency.

<Example 4>

With a THF solution (0.05 wt%) of a phosphorescent compound (MC-1) synthesized by the method described in WO02/06655 and represented by the following formula:

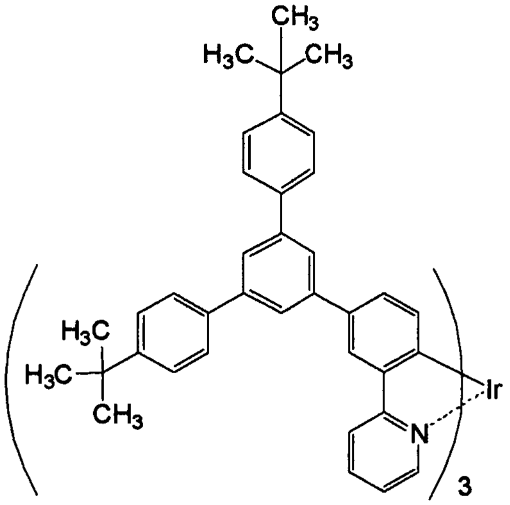

(MC-1)

about a 5-fold weight of a THF solution (about 1 wt%) of a compound (C-3) represented by the following formula:

[Formula 20]

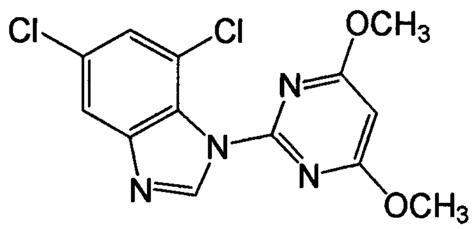

(C-3)

was mixed to prepare a mixture (solution). This mixture (10 μl) was added dropwise to a slide glass and air-dried to obtain a solid film. When the solid film was irradiated with UV rays of 365 nm, strong green light was emitted from the phosphorescent compound (MC-1). From this, it was confirmed that the luminous efficiency of the mixture was high. The $T_1$ energy value of the compound (C-3) was 3.4 eV and the absolute value of an LUMO energy level ($E_{LUMO}$) was 1.8 eV Parameters were calculated by the computational scientific approach in the same manner as in Example 1. Furthermore, the $T_1$ energy value of the phosphorescent compound (MC-1) calculated by the computational scientific approach was 2.7 eV

<Example 5>

A mixture (solution) was prepared in the same manner as in Example 4 except that the phosphorescent compound (MC-1) in Example 4 was replaced with a phosphorescent compound (MC-2) represented by the following formula:

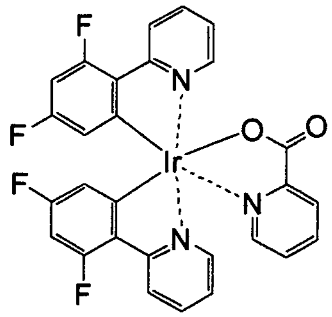

(MC-2)

When the resultant solid film was irradiated with UV rays of 365 nm, intensive light was emitted from the phosphorescent compound (MC-2, trade name: ADS065BE manufactured by American Dye Source, Inc.). From this, it was confirmed that the luminous efficiency of the mixture is high.
The $T_1$ energy value of the phosphorescent compound (MC-2) calculated by the computational scientific approach was 2.9 eV

<Example 6>

With a THF solution (0.05 wt%)of the phosphorescent compound (MC-1), an about 5-fold weight of a THF solution (about 1 wt%) of a compound (C-4) represented by the following formula:

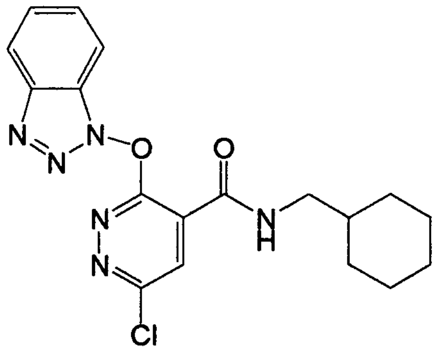

(C-4)

was mixed to prepare a mixture (solution). The mixture (10 μl) was added dropwise to a slide glass and air-dried to obtain a solid film. When the solid film was irradiated with UV rays of 365 nm, strong green light was emitted from the phosphorescent compound (MC-1). From this, it was confirmed that the luminous efficiency of the mixture is high.
As the absolute value of an LUMO energy level ($E_{LUMO}$) of the compound (C-4) was calculated according to the computational scientific approach in the same manner as in Example 1, it was 3.0 eV

<Example 7>

With a THF solution (0.05 wt%)of the phosphorescent compound (MC-1), an about 5-fold weight of a THF solution (about 1 wt%) of a compound (C-5) represented by the following formula:

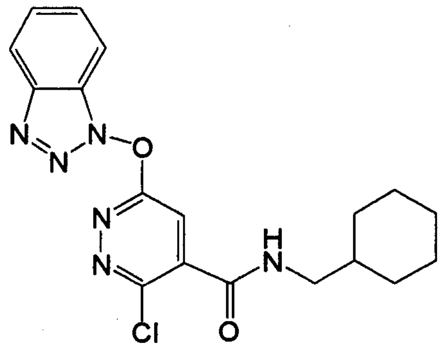

(C-5)

was mixed to prepare a mixture (solution). The mixture (10 μl) was added dropwise to a slide glass and air-dried to obtain a solid film. When the solid film was irradiated with UV rays of 365 nm, strong green light was emitted from the phosphorescent compound (MC-1). From this, it was confirmed that the luminous efficiency of the mixture is high.

As the absolute value of the LUMO energy level ($E_{LUMO}$) of the compound (C-5) was calculated according to the computational scientific approach in the same manner as in Example 1, it was 2.9 eV

<Comparative Example 1>

A polymer compound (CP-1) represented by the following formula:

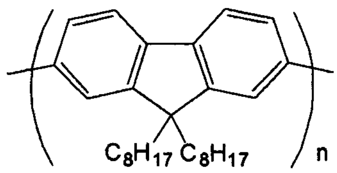

(CP-1)

wherein n is the number of polymerization units,
had a $T_1$ energy value of 2.6 eV, an absolute value of an LUMO energy level ($E_{LUMO}$) of 2.1 eV, and an absolute value of an HOMO energy level ($E_{HOMO}$) of 5.7 eV, which were extrapolation values at n = ∞, and the smallest dihedral angle of 45°. Parameters were calculated by the computational scientific approach in the same manner as in Example 3 by simplifying a repeating unit (CM-1) represented by the following formula (CM-1) in the polymer compound (CP-1) as shown in the following formula (CM-1a).

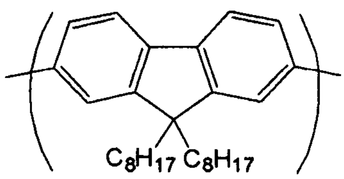

(CM-1)

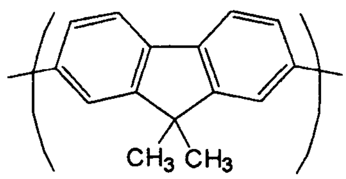

(CM-1a)

Subsequently, a solution mixture (10 μl) containing the polymer compound (CP-1) and the phosphorescent compound (MC-1) was prepared, added dropwise to a slide glass and air-dried to obtain a solid film. When the solid film was irradiated with UV rays of 365 nm, weak light was emitted from the phosphorescent compound (MC-1). From this, it was confirmed that the luminous efficiency of the mixture is low.

INDUSTRIAL APPLICABILITY

The composition, etc. of the present invention can be used for preparing a light-emitting device having excellent luminous efficiency.

## Claims

1. A composition comprising: a compound having a residue(s) of at least one nitrogen-containing compound selected from the group consisting of nitrogen-containing compounds represented by formulas (1-1), (1-2), (1-3) and (1-4) given below:

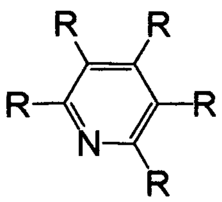
(1-1)

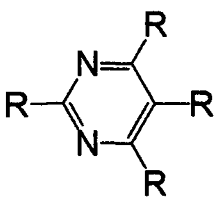
(1-2)

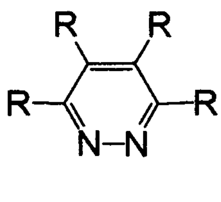
(1-3)

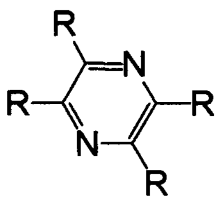
(1-4)

wherein each R represents a hydrogen atom or a substituent; and more than one R may be the same or different, and a residue(s) of at least one nitrogen-containing polycyclic compound selected from the group consisting of nitrogen-containing polycyclic compounds represented by formulas (2-1), (2-2), (2-3) and (2-4) given below:

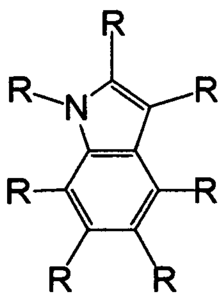
(2-1)

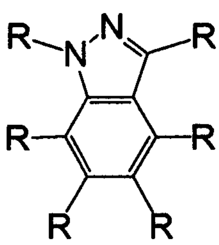
(2-2)

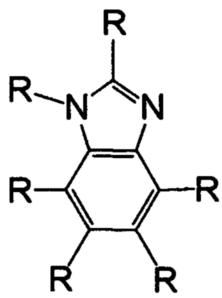
(2-3)

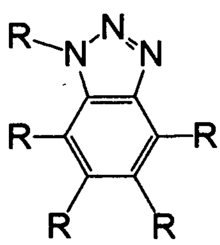
(2-4)

wherein each R is as defined above;
and a phosphorescent compound.

2. The composition according to claim 1, wherein the compound having a residue(s) of a nitrogen-containing compound and a residue(s) of a nitrogen-containing polycyclic compound is a compound having a residue(s) of at least one nitrogen-containing compound selected from the group consisting of nitrogen-containing compounds represented by the formulas (1-2), (1-3) and (1-4) above and a residue(s) of at least one of a nitrogen-containing polycyclic compound selected from the group consisting of nitrogen-containing polycyclic compounds represented by the formulas (2-1), (2-2) and (2-3) given above.

3. The composition according to claim 1 or 2, wherein at least one of the R is an alkyl group, an alkoxy group, an aryl group that may have a substituent or a heteroaryl group that may have a substituent.

4. The composition according to claim 3, wherein at least one of the R is a substituent having 3 or more atoms in total except hydrogen.

5. The composition according to claim 4, wherein at least one of the R is an alkyl group having 3 to 10 carbon atoms or an alkoxy group having 3 to 10 carbon atoms.

6. The composition according to any one of claims 1 to 5, wherein the compound having a residue(s) of a nitrogen-containing compound and a residue(s) of a nitrogen-containing polycyclic compound is a compound represented by formula (A-1) or (A-2) given below:

$$Z^1 — (Y^1)_m — Z^2 \qquad (A\text{-}1)$$

$$Z^1 — (Y^2)_n — Z^2 \qquad (A\text{-}2)$$

wherein one of $Z^1$ and $Z^2$ represents a residue(s) of a nitrogen-containing compound represented by the above formula (1-1), (1-2), (1-3) or (1-4) and the other represents a residue(s) of a nitrogen-containing polycyclic compound represented by the above formula (2-1), (2-2), (2-3) or (2-4); $Y^1$ represents $-C(R^a)(R^b)-$, $-N(R^c)-$, -O-, $-Si(R^d)(R^e)-$, $-P(R^f)-$ or -S-; $R^a$ to $R^f$ each independently represent a hydrogen atom or a substituent; m is an integer of 0 to 5; when there is more than one $Y^1$, they may be the same or different; $Y^2$ represents an arylene group that may have a substituent; n is an integer of 1 to 5; and when there is more than one $Y^2$, they may be the same or different, or a compound having a residue(s) of the foregoing compound.

7. The composition according to any one of claims 1 to 6, wherein the compound having a residue(s) of a nitrogen-containing compound and a residue(s) of a nitrogen-containing polycyclic compound is a polymer.

8. The composition according to claim 7, wherein the compound having a residue(s) of a nitrogen-containing compound and a residue(s) of a nitrogen-containing polycyclic compound is a polymer having a repeating unit comprising a residue(s) of a compound represented by the above formula (A-1) or (A-2).

9. The composition according to any one of claims 1 to 8, wherein the lowest triplet excitation energy of the compound having a residue(s) of a nitrogen-containing compound and a residue(s) of a nitrogen-containing polycyclic compound as calculated by a computational scientific approach is 3.0 eV or more.

10. The composition according to any one of claims 1 to 8, wherein the absolute value of the lowest unoccupied molecular orbital energy level of the compound having a residue(s) of a nitrogen-containing compound and a residue(s) of a nitrogen-containing polycyclic compound as calculated by a computational scientific approach is 1.5 eV or more.

11. The composition according to any one of claims 1 to 8, wherein the absolute value of the highest occupied molecular orbital energy level of the compound having a residue(s) of a nitrogen-containing compound and a residue(s) of a nitrogen-containing polycyclic compound as calculated by a computational scientific approach is 6.2 eV or less.

12. The composition according to any one of claims 1 to 8, wherein the lowest triplet excitation energy value (ETH) of the compound having a residue(s) of a nitrogen-containing compound and a residue(s) of a nitrogen-containing polycyclic compound and the lowest triplet excitation energy value (ETG) of the phosphorescent compound satisfy the expression given below:

$$ETH > ETG\ (eV).$$

13. The composition according to any one of claims 7 to 12, wherein the compound having a residue(s) of a nitrogen-containing compound and a residue(s) of a nitrogen-containing polycyclic compound is a compound having a heterocyclic structure constituting the nitrogen-containing compound and the nitrogen-containing polycyclic compound and a partial structure adjacent to the heterocyclic structure, the partial structure having at least two $\pi$-conjugated electrons, wherein the dihedral angle between the heterocyclic structure and the partial structure is 40° or more.

**14.** The composition according to any one of claims 1 to 13, wherein the phosphorescent compound is an iridium complex or a platinum complex.

**15.** The composition according to claim 14, wherein the phosphorescent compound is a metal complex having iridium or platinum as a central metal and having 8-quinolinol or a derivative thereof, benzoquinolinol or a derivative thereof, or 2-phenyl-pyridine or a derivative thereof as a ligand.

**16.** A polymer comprising: a residue(s) of at least one nitrogen-containing compound selected from the group consisting of nitrogen-containing compounds represented by formulas (1-1), (1-2), (1-3) and (1-4) given below:

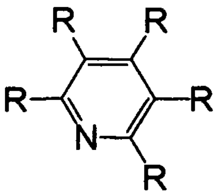
(1-1)

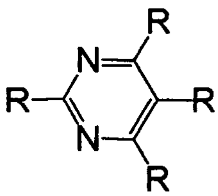
(1-2)

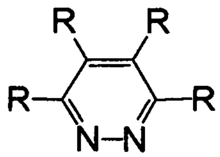
(1-3)

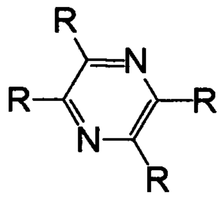
(1-4)

wherein R represents a hydrogen atom or a substituent; and more than one R may be the same or different;
a residue(s) of at least one nitrogen-containing polycyclic compound selected from the group consisting of nitrogen-containing polycyclic compounds represented by formulas (2-1), (2-2), (2-3) and (2-4) given below:

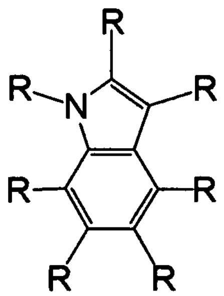
(2-1)

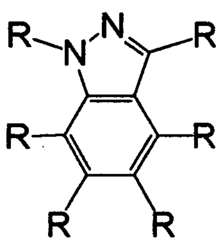
(2-2)

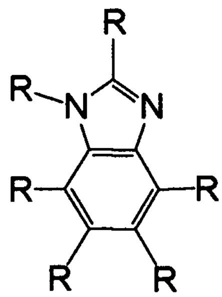
(2-3)

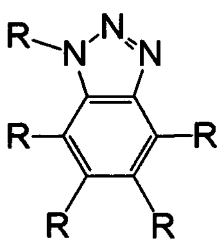
(2-4)

wherein R is as defined above;
and a residue of a phosphorescent compound.

**17.** A film prepared by using the composition according to any one of claims 1 to 15 or the polymer according to claim 16.

**18.** A light-emitting device prepared by using the composition according to any one of claims 1 to 15 or the polymer according to claim 16.

**19.** A planar light source comprising the light-emitting device according to claim 18.

**20.** A display comprising the light-emitting device according to claim 18.

**21.** A light comprising the light-emitting device according to claim 18.

**INTERNATIONAL SEARCH REPORT**

International application No.
PCT/JP2009/061365

A. CLASSIFICATION OF SUBJECT MATTER
*H01L51/50*(2006.01)i, *C07D403/04*(2006.01)i, *C07D403/14*(2006.01)i, *C07F15/00*(2006.01)i, *C08G61/12*(2006.01)i, *C08G73/06*(2006.01)i, *C08K5/34*(2006.01)i, *C08K5/56*(2006.01)i, *C08L65/00*(2006.01)i, *C09K11/06*(2006.01)i
According to International Patent Classification (IPC) or to both national classification and IPC

B. FIELDS SEARCHED
Minimum documentation searched (classification system followed by classification symbols)
H01L51/50, C07D403/04, C07D403/14, C07F15/00, C08G61/12, C08G73/06, C08K5/34, C08K5/56, C08L65/00, C09K11/06

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br><br>A | JP 2004-031004 A (Konica Minolta Holdings, Inc.),<br>29 January, 2004 (29.01.04),<br>Claims; Par. Nos. [0039] to [0061], [0095]; examples<br>(Family: none) | 6,14,15,<br>17-21<br>7,8 |
| X<br><br>A | JP 2005-082701 A (Toyo Ink Manufacturing Co., Ltd.),<br>31 March, 2005 (31.03.05),<br>Claims; Par. Nos. [0023] to [0069], [0071] to [0074], [0087]; examples<br>(Family: none) | 6,14,15,<br>17-21<br>7,8 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>14 August, 2009 (14.08.09) | Date of mailing of the international search report<br>25 August, 2009 (25.08.09) |
| Name and mailing address of the ISA/<br>Japanese Patent Office<br>Facsimile No. | Authorized officer<br><br>Telephone No. |

Form PCT/ISA/210 (second sheet) (April 2007)

**INTERNATIONAL SEARCH REPORT**

International application No.
PCT/JP2009/061365

C (Continuation). DOCUMENTS CONSIDERED TO BE RELEVANT

| Category* | Citation of document, with indication, where appropriate, of the relevant passages | Relevant to claim No. |
|---|---|---|
| X<br><br>A | JP 2003-277743 A (Idemitsu Kosan Co., Ltd.), 02 October, 2003 (02.10.03), Claims; Par. Nos. [0012] to [0015], [0018], [0030]; examples (Family: none) | 6,14,15, 17-21<br>7,8 |
| X<br><br>A | JP 2008-133225 A (Toyo Ink Manufacturing Co., Ltd.), 12 June, 2008 (12.06.08), Claims; Par. Nos. [0093] to [0142], [0167], [0174]; examples (Family: none) | 6,14,15, 17-21<br>7,8 |
| P,X | WO 2008/096736 A1 (Sumitomo Chemical Co., Ltd.), 14 August, 2008 (14.08.08), Claims; Par. Nos. [0039] to [0048], [0063] to [0067]; examples & JP 2008-218986 A | 6-8,14,15, 17-21 |
| A | CN 1562999 A (Shanghai Institute of Organic Chemistry, Chinese Academy of Sciences, Peop. Rep. China), 12 January, 2005 (12.01.05), Claims; examples (Family: none) | 6-8,14,15, 17-21 |

Form PCT/ISA/210 (continuation of second sheet) (April 2007)

**INTERNATIONAL SEARCH REPORT**

International application No.
PCT/JP2009/061365

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☒ Claims Nos.: 1-5,9-13,16
because they relate to parts of the international application that do not comply with the prescribed requirements to such an extent that no meaningful international search can be carried out, specifically:
See extra sheet.

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** the

☐ The additional search fees were accompanied by the applicant's protest and, where applicable, payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2007)

INTERNATIONAL SEARCH REPORT

International application No.
PCT/JP2009/061365

Continuation of Box No.II-2 of continuation of first sheet(2)

In the inventions of claims 1-5, a specific compound is defined by its partial structure and, therefore, a person skilled in the art cannot specify the scope of compounds included within the definition of the specific compound.

In the inventions of claims 9-13, a specific compound or polymer is defined by the results of the calculation by a computational science procedure and, therefore, a person skilled in the art cannot specify the scope of compounds or polymers included within the definition of the specific compound or polymer.

In the invention of claim 16, a polymeric compound having specific residues and a residue of a phosphorescent light-emitting compound is defined. However, it cannot be considered that the polymeric compound is fully supported by the description or are clearly and completely described to such an extent that a person skilled in the art can put the invention into practice.

<Subject of search>

The inventions of claims 6-8 define many compounds or polymers each having any one of all available choices of the combination of a residue of a nitrogen compound and a residue of a nitrogenated polycyclic compound. However, the description discloses only embodiments having an extremely small part of the available choices of the combination of the residues.

Further, it is not necessarily obvious for a person skilled in the art that all of the compounds or polymers represented by the general formula can be used as compounds for organic EL elements in the light of the common technical knowledge in the art.

With regard to the claimed inventions which are not fully supported by the description and are not disclosed clearly or completely in the description, the search was not made.

Form PCT/ISA/210 (extra sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2002050483 A
- JP 2002241455 A
- WO 2002066552 A
- WO 2004020504 A
- WO 2004020448 A
- JP 63070257 A
- JP 63175860 A
- JP 2135359 A
- JP 2135361 A
- JP 2209988 A
- JP 3037992 A
- JP 3152184 A
- JP 2005126686 A
- WO 0206655 A


### Non-patent literature cited in the description


- *APPLIED PHYSICS LETTERS,* 2002, vol. 80 (13), 2308
- *Nature,* 1998, vol. 395, 151
- *Appl. Phys. Lett.,* 1999, vol. 75 (1), 4
- Organic Light-Emitting Materials and DevicesIV. *Proc. SPIE-Int. Soc. Opt. Eng.,* 2001, vol. 4105, 119
- *J. Am. Chem. Soc.,* 2001, vol. 123, 4304
- *Appl. Phys. Lett.,* 1997, vol. 71 (18), 2596
- *Syn. Met.,* 1998, vol. 94 (1), 103
- *Syn. Met.,* 1999, vol. 99 (2), 1361
- *Adv. Mater.,* 1999, vol. 11 (10), 852
- *Inorg. Chem.,* 2003, vol. 42, 8609
- *Inorg. Chem.,* 2004, vol. 43, 6513
- *Journal of the SID 11/1,* 2003, 161